# EUROPÄISCHE PATENTSCHRIFT

(11) **EP 0 423 383 B1**
(45) Veröffentlichungstag und Bekanntmachung des Hinweises auf die Patenterteilung: **08.09.1993**
(21) Anmeldenummer: 89119178.5
(22) Anmeldetag: 16.10.1989
(51) Int. Cl.: H05G 1/54, A61B 6/10

(54) **Röntgeneinrichtung**
X-ray apparatus
Appareil de radiologie

(43) Veröffentlichungstag der Anmeldung: 24.04.1991
(73) Patentinhaber: SIEMENS AKTIENGESELLSCHAFT, 80333 München (DE)
(72) Erfinder: Gerlach, Hans-Jürgen, Dipl.-Ing., D-8520 Erlangen (DE); Reimer, Jürgen, Dipl.-Ing., D-8520 Erlangen (DE)

(56) Entgegenhaltungen:
- EP-A- 0 092 705
- DE-C- 973 690
- FR-A- 2 431 239
- GB-A- 2 069 129

## Beschreibung

Bekannte Röntgeneinrichtungen weisen eine Kontrolleuchte auf, die anzeigt, wenn die Röntgenröhre zur Aussendung von Röntgenstrahlung eingeschaltet ist (siche zum Beispiel FR-A-2 431 239). Dabei ist jedoch eine fehlerhafte Anzeige in der Weise möglich, daß die Kontrolleuchte leuchtet, obwohl keine Strahlung auftritt, z.B. infolge eines schadhaften Schaltschützes, oder daß trotz des Auftretens von Strahlung die Kontrolleuchte infolge eines Defekts nicht anzeigt.

Der Erfindung liegt die Aufgabe zugrunde, eine Röntgeneinrichtung zu schaffen, bei der mit Sicherheit das Auftreten von Strahlung angezeigt wird.

Diese Aufgabe ist erfindungsgemäß durch eine Röntgeneinrichtung gelöst, wobei im Blendengehäuse der Primärstrahlenblende oder im Gehäuse des Röntgenstrahlers eine Leuchtfolie angeordnet ist, deren Licht durch einen nach außen führenden Lichtwellenleiter zur Anzeige übertragen wird. Das äußere Ende des Lichtwellenleiters kann dabei geringfügig aus dem Blendengehäuse bzw. Strahlergehäuse herausragen, so daß von außen das Auftreten von Strahlung erkennbar ist. In diesem Fall leuchtet nämlich die Leuchtfolie auf, was durch den Lichtwellenleiter nach außen übertragen wird.

Es ist möglich, einen einzigen Lichtwellenleiter vorzusehen und das Licht der Leuchtfolie an eine bestimmte Stelle des Blenden- oder Strahlergehäuses zu übertragen, es ist aber auch möglich, dieses Licht über mehrere Lichtwellenleiter zu verschiedenen Stellen am Blendengehäuse bzw. Strahlergehäuse zu übertragen, so daß aus jeder Position heraus leicht überprüfbar ist, ob Strahlung auftritt oder nicht.

In den Fällen, in denen der Röntgenstrahlenerzeuger (Strahler mit Blende) - verdeckt durch andere Teile der Einrichtung - nicht sichtbar angebracht ist, kann auch durch geeignete Ausbildung der Lichtleiterübertragungsstrecke der Betrachtungsort an eine hierfür geeignete Position verlegt werden.

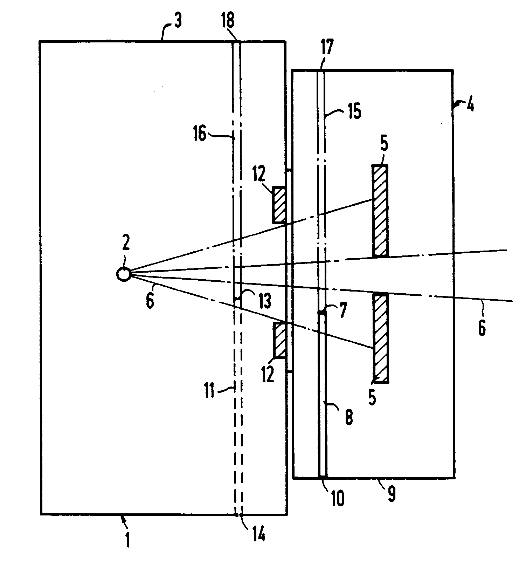

Die Erfindung ist nachfolgend anhand eines in der Zeichnung dargestellten Ausführungsbeispieles näher erläutert.

In der Zeichnung ist ein Röntgenstrahler 1 mit einem Fokus 2 dargestellt, an dessen Strahlenaustrittsfenster 12 eine Primärstrahlenblende 4 angebracht ist. Blendenplatten 5 begrenzen das austretende Röntgenstrahlenbündel 6.

Im Innern der Primärstrahlenblende 4 ist eine Leuchtfolie 7 angeordnet, die durch Röntgenstrahlung zum Leuchten angeregt wird und den bildgebenden Strahlengang nicht beeinträchtigt. Die Leuchtfolie 7 kann in gleicher Weise wie die bekannten Verstärkerfolien in Röntgenfilmkassetten ausgebildet sein. Das Licht der Leuchtfolie 7 wird über einen Lichtwellenleiter 8 nach außen zum Gehäuse 9 geführt. Wenn das äußere Ende 10 des Lichtwellenleiters 8, das von außen sichtbar ist, leuchtet, ist dies ein Zeichen für das Auftreten von Röntgenstrahlung.

Bei der dargestellten Röntgeneinrichtung ist es mit großer Sicherheit möglich, das Auftreten von Röntgenstrahlung zu überwachen, da hierzu nicht abgeleitete Größen, wie z.B. die Spannung an der Röntgenröhre, überwacht werden, sondern die Strahlung selbst.

In der Zeichnung ist gestrichelt eingezeichnet, daß ein dem Lichtwellenleiter 8 entsprechender Lichtwellenleiter 11 mit einer Leuchtfolie 13 anstelle der Komponenten 7, 8 im Gehäuse 3 des Röntgenstrahlers 1 angeordnet sein kann. Am äußeren Ende 14 des Lichtwellenleiters 11 kann dann das Auftreten von Röntgenstrahlung beobachtet werden.

Das Licht der Leuchtfolie 7, 13 kann anstelle zu den Außenseiten des Gehäuses 9 bzw. des Gehäuses 3 des Röntgenstrahlers 1 auch weiter zu einem geeigneten Betrachtungsort übertragen werden.

Strichpunktiert sind noch Lichtwellenleiter 15, 16 eingezeichnet, die das Licht der Leuchtfolie 7, 13 zu den Stellen 17, 18 am Blendengehäuse 9 bzw. Strahlerghäuse 3 übertragen. Auf diese Weise ist von mehreren Positionen aus das Auftreten von Strahlung überwachbar.

## Patentansprüche

1. Röntgeneinrichtung mit einer Primärstrahlenblende (4), einer im Blendengehäuse (9) angeordneten Leuchtfolie (7) und einem das Licht der Leuchtfolie (7) zur Anzeige nach außen führenden Lichtwellenleiter (8).

2. Röntgeneinrichtung mit einer im Gehäuse (3) des Röntgenstrahlers (1) angeordneten Leuchtfolie (13) und einem das Licht der Leuchtfolie (13) zur Anzeige nach außen führenden Lichtwellenleiter (11).

3. Röntgeneinrichtung nach Anspruch 1 oder 2, **dadurch gekennzeichnet,** daß mehrere Lichtwellenleiter (8, 15; 11, 16) im Blendengehäuse (9) bzw. Strahlergehäuse (3) vorhanden sind, die das Licht der Leuchtfolie (7, 13) zu verschiedenen Stellen (10, 17; 14, 18) übertragen.

## Claims

1. X-ray apparatus having a primary radiation diaphragm (4), a luminescent foil (7) arranged in the diaphragm housing (9) and an optical waveguide (8) which conducts the light from the luminescent foil (7) towards the exterior for display purposes.

2. X-ray apparatus having a luminescent foil (13) arranged in the housing (3) of the X-ray source (1) and an optical waveguide (11) which conducts the light from the luminescent foil (13) towards the exterior for display purposes.

3. X-ray apparatus according to claim 1 or 2, characterised in that several optical waveguides (8, 15; 11, 16) are present in the diaphragm housing (9) and X-ray source housing (3) respectively, for transmitting the light from the luminescent foil (7, 13) to various locations (10, 17; 14, 18).

## Revendications

1. Appareil radiologique comportant un diaphragme du rayonnement primaire (4), une feuille luminescente (7) disposée dans le boîtier (9) du diaphragme et un guide d'ondes optiques qui transmet la lumière de la feuille luminescente (7) vers l'extérieur en vue de son affichage.

2. Appareil radiologique comportant une feuille luminescente (13) disposée dans le boîtier (3) de l'émetteur radiologique (1), et un guide d'ondes optiques qui transmet la lumière de la feuille luminescente (13) vers l'extérieur pour son affichage.

3. Dispositif radiologique suivant la revendication 1 ou 2, caractérisé par le fait que dans le boîtier (9) du diaphragme ou dans le boîtier (3) de l'émetteur, il est prévu plusieurs guides d'ondes optiques (8,15;11,16), qui transmettent la lumière de la feuille luminescente (7,13) en différents emplacements (10,17;14,18).
